# EUROPEAN PATENT APPLICATION

(11) **EP 2 832 321 A1**
(43) Date of publication of application: **04.02.2015**
(21) Application number: 13178983.6
(22) Date of filing: 01.08.2013
(51) Int. Cl.: A61F 2/42, A61F 2/30

(54) **Implant system for total ankle replacement**

(71) Applicant: Universitätsspital Basel, 4031 Basel (CH)
(72) Inventor: Nowakowski, Andrej, CH-4147 Aesch (CH); Iselin, Lukas Daniel, CH-4125 Riehen (CH)
(74) Representative: Schulz, Ben Jesko

(57) **Abstract**

Implant system for a total ankle replacement, comprising: a tibial implant (1) being designed to be fastened to a distal end (4) of the tibia (T), as well as particularly an intermediate implant (2) and a talar implant (3). According to the invention a separate locking device (11) is provided being designed to be inserted into a first recess (R) of the tibia (T) extending along said distal end (4) of the tibia (T), wherein the tibial implant (1) comprises a peg (12) protruding from a base (1a) of the tibial implant (1), which peg (12) is designed to be inserted into a second recess (R') in said distal end (4) of the tibia (T), which second recess (R') particularly communicates with the first recess (R'), and wherein when the locking device (11) is inserted into the first recess (R) and the peg (12) is inserted into the second recess (R') the locking device (11) is designed to fasten the peg (12) and thereby the tibial implant (1) to the locking device (11).

## Description

The invention relates to an implant system for a total ankle replacement according to the features of claim 1.

Such systems usually comprise a tibial implant being designed to be fastened to a distal end of the tibia. Such a tibial implant is also denoted as tibial plafond plate.

Such an implant system can further comprise an intermediate implant also denoted as inlay and a talar implant also denoted as talus cap to be implanted in or on the talus.

Total ankle replacements in humans are used to preserve movement in damaged ankle joints due to injury or disease (e.g. rheumatoid arthritis, tumors etc.).

Several implant designs are known such as e.g. EP 1 097 680 B1, EP 1 482 874 B1, EP 1433 444 B1 and EP 1 731 115 B1.

The aseptic loosening of total ankle replacements being a consequence of mechanical loosening e.g. due to a lack of ingrowth into the bone or due to particle deposition occurs more often compared to the well established hip and knee replacements where stability is a lesser issue.

There are different types of tibial fixation. E.g., the implant is only underlying the distal tibia and additionally fixed with screws via an anterior shield (cf. EP 1 097 680 B1); other implants may need a disruption of the anterior cortex in order to introduce the stem.

In order to achieve primary stability in total ankle replacements, the tibial plafond plate has to be tightly fixed against the tibial bone. One of the main contributors to stability is the maintenance of the integrity of the circular cortical structure.

The problem underlying the present invention is therefore to provide for an implant system for a total ankle replacement that achieves an improved implant stability, particularly in order to provide for a better secondary osseous integration.

This problem is solved by an implant system comprising the features of claim 1.

Preferred embodiments are stated in the sub claims.

According to claim 1 a separate locking device is provided being designed to be inserted into a first recess of the tibia extending along said distal end of the tibia, wherein the tibial implant comprises a peg protruding from a base of the tibial implant, which peg is designed to be inserted into a second recess in said distal end of the tibia, which second recess communicates with the first recess, and wherein - when the locking device is inserted into the first recess and the peg is inserted into the second recess - said locking device is designed to fasten the peg to the locking device and thereby the tibial implant to the distal end of the tibia.

Preferably, when the locking device is inserted into the first recess and the peg is inserted into the second recess, the locking device is designed to pull the peg and thereby the tibial implant towards the locking device so as to press the tibial implant against said distal end of the tibia.

Thus, advantageously, the invention allows for tightly fixing the tibial implant against said distal end of the tibia.

According to a preferred embodiment of the present invention, the locking device is designed to receive an end portion of the peg for fastening the peg to the locking device and/or for pulling the peg towards the locking device when the locking device is inserted into the first recess and the peg is inserted into the second recess.

Further, according to a preferred embodiment of the present invention, the locking device comprises a rotatable element being designed to be rotated between a first and a second position about a rotation axis, wherein preferably upon rotation of the rotatable element from the first towards the second position, the rotatable element is preferably designed to engage behind said end portion received by the locking device and to thereby pull said end portion towards the locking device, so as to press the tibial implant against said distal end of the tibia.

Further, according to a preferred embodiment of the present invention, the locking device comprises a tubular outer shell having a lateral aperture for insertion of said end portion of the peg into the locking device.

Further, according to a preferred embodiment of the present invention, the rotatable element is rotatably mounted inside said outer shell about said rotation axis.

For this, according to a preferred embodiment of the present invention, the rotatable element is formed as a particularly tubular inner shell that is particularly coaxially arranged with respect to the outer shell in said outer shell. The inner shell may have sufficient play inside the outer shell so that the inner shell can be rotated with respect to the outer shell, wherein an outer side of a circumferential wall of the inner shell slides along an inner side of a circumferential wall of the outer shell. Preferably, said shells have a cylindrical shape. Further, the e.g. common axis of the shells is a longitudinal axis (e.g. cylinder axis) of the shells. Particularly, said longitudinal axis coincides with said rotation about which the inner shell can be (e.g. manually) rotated.

Further, according to a preferred embodiment of the present invention, said rotatable inner shell comprises a lateral aperture in said circumferential wall of the inner shell for receiving said end portion of the peg.

Further, according to a preferred embodiment of the present invention, for allowing the locking device to receive the end portion of the peg, said lateral aperture of said rotatable inner shell aligns with the lateral aperture of the outer shell in said first position of the rotatable inner shell, so that said end portion of the peg can be inserted through both aligned apertures into the locking device, i.e., into the inner shell residing in the outer shell.

Further, according to a preferred embodiment of the present invention, the rotatable inner shell comprises a slot formed in said wall of the inner shell, which slot is connected to the lateral aperture and extends starting from said aperture of the inner shell along a peripheral direction of the inner shell (which peripheral direction runs around the inner shell across the longitudinal/rotation axis of the inner shell) and is delimited by opposing regions of said wall of the inner shell. Preferably, the rotatable inner shell is designed to engage behind said end portion of the peg with said regions/edges of the wall delimiting said slot upon rotation from the first position into the second position.

Further, according to a preferred embodiment of the present invention, the thickness of said wall regions delimiting the slot increases along the edges of the slot in said peripheral direction away from the lateral aperture of the wall of the inner shell, so that upon rotation of the inner shell from the first position into the second position the end portion is continuously pulled further into the locking device as the wall regions engaging behind the end portion of the peg become continuously thicker upon rotation of the inner shell into its second position.

Thus, advantageously, the locking device actually allows for adjusting the force by means of which the tibial implant is pressed against the distal end of the tibia. The further the inner shell is rotated towards the second position the larger said force. Said second position may be defined by an end of the slot that forms a catch for the peg corresponding to a maximum force by means of which the tibial implant is pulled towards the locking device or pressed against the distal end of the tibia.

Preferably, generally, the locking device is designed to be self-locking, i.e., once the inner shell has been rotated into a certain position towards the second position, friction forces prevent the inner shell from rotating back into the initial (e.g. first) position of the inner shell.

According to a preferred embodiment of the present invention, said end portion of the peg is formed as a sphere which is connected via a narrow portion of the peg to a base of the peg, which base of the peg protrudes from the base of said plate so that the peg forms a circumferential groove along said narrow portion, wherein particularly said narrow portion is designed to be received by said slot of the rotatable inner shell when said edges engage behind the (e.g. spherical) end portion of the peg.

Further, preferably said base of the peg comprises a conical shape. Accordingly, the associated second recess is drilled such into the distal end of the tibia that it also comprises a conical shape so that the peg can be form-fittedly inserted into the second recess. When the tibial implant is now pressed against the distal end of the tibia by means of the locking device, comparably large forces are generated normal to the conical inner side of the second recess, so that the tibial implant is very strongly attached to the distal end of the tibia.

Further, according to a preferred embodiment of the present invention, said inner shell comprises a recess in a face side of the inner shell for insertion of a tool for rotating the rotatable inner shell between said first and second position.

Apart from a rotatable element, other fastening means may also be used to fasten the peg to the locking device, for instance, the locking device may comprise a fastening means, particularly in the form of a cone or a wedge, wherein preferably said fastening means interacts with the locking device and the peg such that the peg is pulled towards the locking device so as to press the tibial implant against said distal end of the tibia when the fastening means fastens the peg to the locking device.

Furthermore, the tibial implant preferably comprises a lower surface facing away from the distal tibia, wherein particularly said lower surface is flat.

Further, the implant system preferably comprises a talar implant which is designed to be implanted in or on the talus, wherein the talar implant comprises an upper surface facing towards the tibial implant, wherein particularly said upper surface is a saddle-shaped upper surface.

Further, the implant system preferably comprises an intermediate implant being designed to be arranged between the tibial implant and the talar implant, which intermediate implant comprises an upper surface for contacting said lower surface of the tibial implant, wherein particularly said upper surface is flat, and wherein the intermediate implant preferably comprises a lower surface for contacting the upper surface of the talar implant.

In the special condition of a talus necrosis or a defect after implant removal, the implant system according to the invention also allows for changing the rotation centre of the prosthesis into an extra-anatomical implant. This means that the center of rotation is transferred from distal (in the talus) to proximal. In an anatomical prosthesis, the surfaces are oriented as in the natural default. An extra-anatomical prosthesis inverts the head-pan relations. This can also be done in case of the ankle by placing the convex surface on the tibia and the concave one on the hindfoot, in order to use the latter in case of a large defect.

Commonly, the implantation of a total ankle replacement is done by either a direct anterior approach or a lateral approach. This invention can be implanted through either an anterior or a lateral approach. The locking device comprising said inner and outer shell can be set in an antero-posterior or a latero-medial position.

The components of the implant device such as the tibial implant, the locking device and the talar implant may be formed out of a metal, e.g. steel. The intermediate implant is preferably formed out of a plastic material such as polyethylene.

In the following, embodiments of the present invention shall be described with reference to the Figures, wherein
- Fig. 1: shows an antero-posterior view of a human ankle joint showing a cross section of an implant system for total ankle replacement according to the present invention with an antero-posterior position of the locking device;
- Fig. 2: shows a lateral view of a human ankle joint with an implant system for total ankle replacement;
- Fig. 3: shows an enlarged cross sectional view of the locking device engaging with the peg of the tibial implant of an implant system according to the invention;
- Fig. 4: shows an isometric view of the locking device before insertion of the end portion of the peg into the locking device; and
- Fig. 5: is a lateral view of a human ankle joint with an implant system according to the invention in an extra-anatomical version.

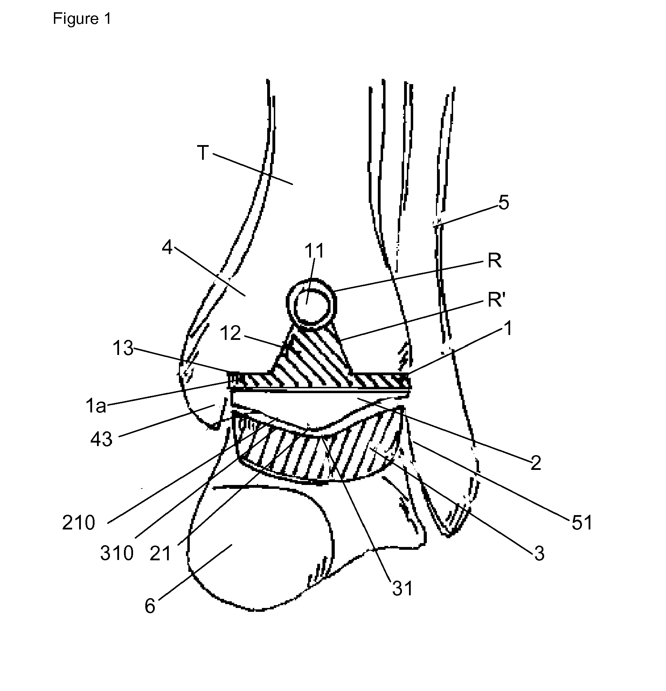

Fig. 1 shows an implant system according to the invention replacing a human ankle joint.

The implant system comprises a tibial implant 1, also denotes as tibial plafond plate, an intermediate implant (also denoted as inlay) 2 and a talar implant (also denoted as talus cap) 3 implanted in or on the talus 6, wherein the intermediate implant 2 is arranged between the tibial implant 1 and the talar implant 3.

The tibial implant 1 is fastened to the distal end 4 of the tibia so that an upper surface 13 of the tibial implant 1 form-fittedly butts against the distal tibial joint surface 42.

The tibial implant 1 comprises a conical central peg 12 protruding from a plate-like base 1a of the tibial implant 1, wherein said peg 12 extends perpendicular to said base 1a of the tibial implant 1 and is inserted into a conical second recess R' drilled into the distal end 4 of the tibia T along the longitudinal axis of the shaft of the tibia T.

The tibial implant 1 is pressed against the distal end 4 of the tibia T by means of an elongated tubular locking device 11 which is inserted into a cylindrical first recess R drilled into into the tibia T and extending along said joint surface 42 of the tibia T in antero-posterior direction, wherein the two recesses R, R' communicate.

The intermediate implant 2 is sitting under the tibial implant 1 and has a suitable lower surface 210 for articulation to an upper surface 310 of the talar implant 3. The upper talar surface 310 can have a saddle-shaped form.

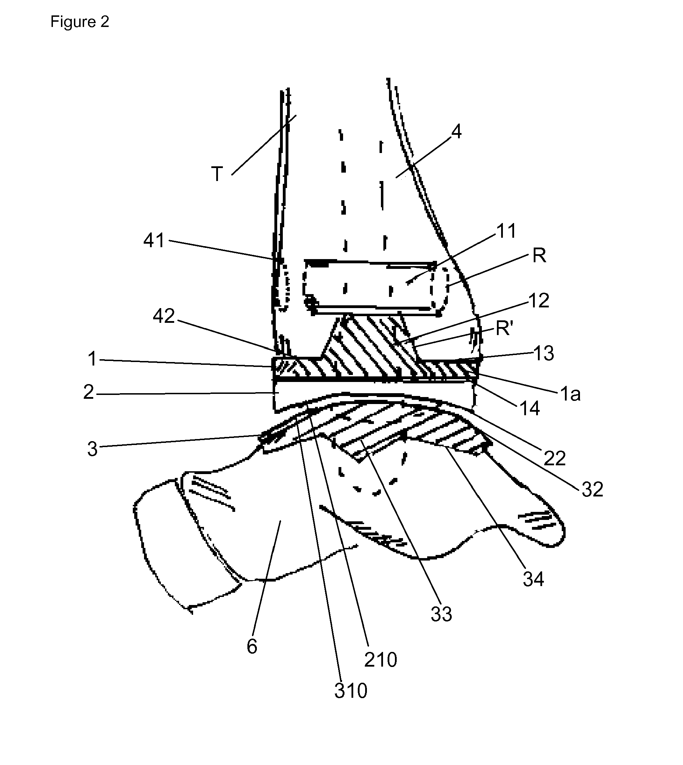

So, correspondingly, the lower surface 210 of the intermediate implant 2 may comprise concave portions 22 laterally (cf. Fig. 2) as well as convex portions 21 antero-posterior (cf. Fig. 1). The upper surface 310 of the talar implant 3 facing the lower surface 210 of the intermediate implant 2 follows the course of the lower surface 210 of the intermediate implant 2. Thus, the upper surface 310 of the talar implant 3 comprises concave portions 31 associated to convex portions 21 of the lower surface 210 of the intermediate implant 2, respectively, as well as convex portions 32 associated to concave portions 22 of the lower surface 210 of the intermediate implant 2. The talar implant 3 may not be guided or guided in order not to impinge in the gutters medially 43 and laterally 51.

Fig. 2 is a lateral view of a human ankle joint with the implant system according to the invention as shown in Fig. 1 showing the tibia T with a drilled first recess (e.g. tunnel/round cut hole) in the anterior cortex 41 for insertion of the tubular locking device 11. The lateral portions 22 of the lower surface 210 of the intermediate implant (inlay) 2 have a radius differing from the radius of the corresponding portions 32 of the upper surface 310 of the talar implant 3.The medial radius is smaller than the lateral radius, so that the talar implant 3 can be tilted with respect to the intermediate implant 2. The intermediate implant 2, which may consist of polyethylene, particularly allows for avoiding a metal on metal contact which may lead to unwanted debris.

The tibial implant 1 can be made from full metal or hollow metal. The tibial implant 1 is specified to be larger on the posterior aspect than on the anterior one. Preferably, it has rounded corners. The lower (articular) surface 14 of the tibial implant can be plain polished in order to allow for a free intermediate implant (inlay) 2 movement. The upper surface 13 of the tibial implant 1 facing the distal end 4 of the tibia T can be supported by additional spikes or other known fixation aids.

The intermediate implant (inlay) 2 can be fixed to the tibial implant 1 or may be mobile/displaceable with respect to the lower (articular) surface 14 of the tibial implant 1. The Inlay 2 can be highly congruent towards the talar implant 3 which means that the contact surface of the inlay 2 towards the talus does essentially not allow for a rotation or merely has little play. Alternatively, the inlay 2 may allow for an inlay rotation (with respect to the tibial implant).

Fig. 1 and 2 illustrate the talar implant (also denoted as talus cap) 3 with its upper (articular) surface 310 polished round (convex 32 to concave 31). It can have two central pegs 33 protruding from a lower side of the talar implant 3 facing away from said upper (articular) surface 310. The anterior aspect of the talar implant 3 seated on the talus 6 can have a prolongation shield which can be used for screw fixation through screw-holes. The talar implant 3 can be fixed to the talus 6 by means of a pressfit technique and/or by means of screws to increase primary stability. There is a possibility to cross-fix the subtalar joint (talo-calcaneal arthrodesis) with screws. As mentioned with respect to the bone facing upper surface 13 of the tibial implant, the lower surface 34 of the talar implant 3 can be covered with usual surface coatings and supporting aids.

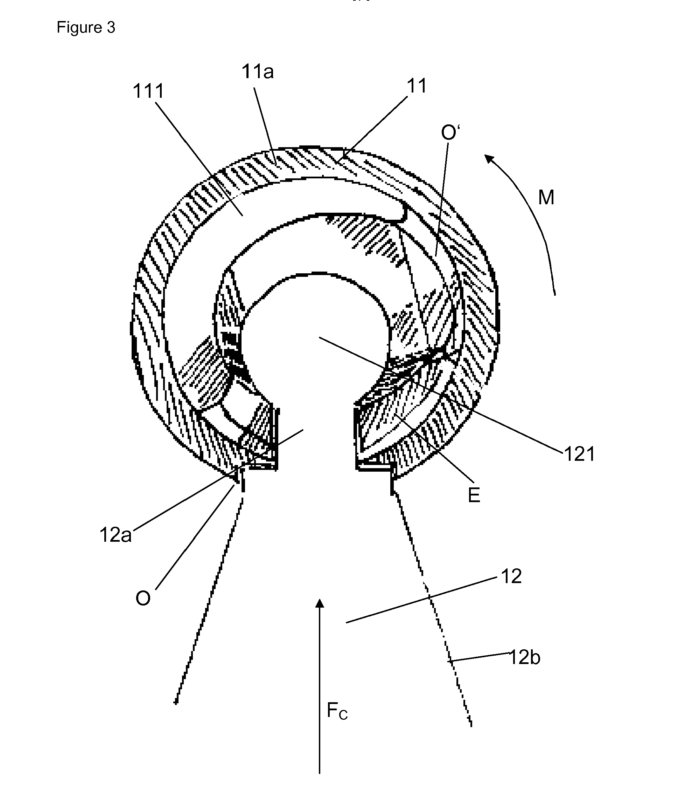

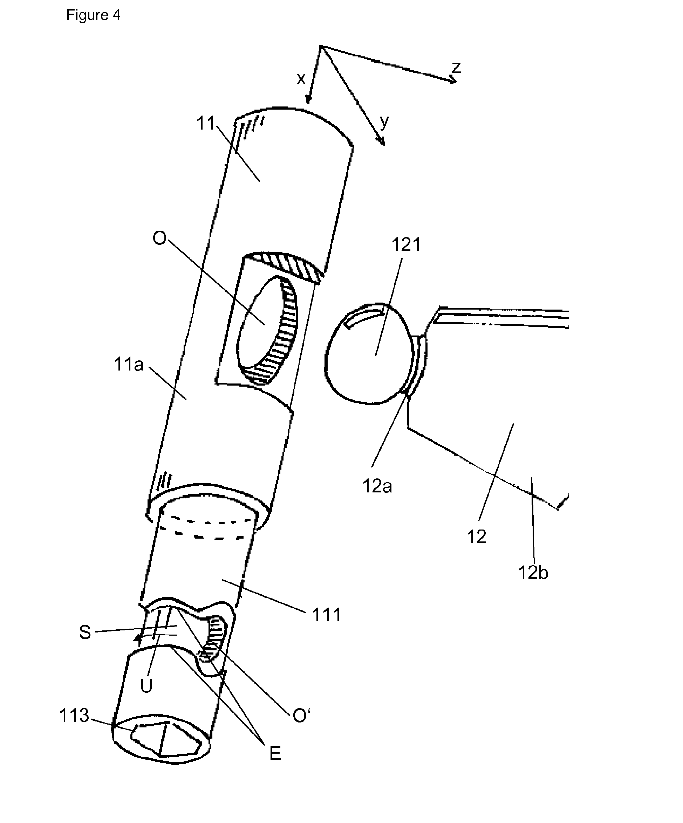

Fig. 3 shows in conjunction with Fig. 4 the locking device 11 that allows to generate a compression force F_{c} by means of which the tibial implant 1 is pressed against the distal end 4 of the tibia T.

The locking device 11 comprises a cylindrical outer shell 11a having a lateral aperture O for receiving a spherical end portion 121 of the peg 12, which is connected via a narrow portion 12a of the peg 12 to a conical base 12b of the peg 12 which in turn protrudes from the base 1a of the tibial implant 1.

Inside said outer shell 11 a, a rotatable element in the form of a cylindrical inner shell 111 is rotatably mounted, so that the inner shell 111 can be rotated with respect to the outer shell 11a about a rotation axis x (in direction M) which coincides with the longitudinal axes x of the shells 11a, 111. The shells 11a, 111 are arranged coaxially with respect to each other.

The rotatable inner shell 111 also comprises a lateral aperture O' in a circumferential wall of the inner shell 111 for receiving said end portion 121 of the peg 12.

Fig. 4 shows the inner shell 111 in an advanced position. However, when fastening the tibial implant 1 by means of the locking device 11 the inner shell 111 is fully inserted into the outer shell 11 so that the lateral apertures O, O' can be aligned in a first position of the inner shell, so that the end portion 121 of the peg 12 can be inserted into the inner shell 111 through both aligned apertures O, O'.

The rotatable inner shell 111 now comprises a slot S formed in said wall of the inner shell 111, which slot S extends starting from said aperture O' of the inner shell 111 along a peripheral direction U of the inner shell 111 and is delimited by opposing wall regions E of said wall. Further, the inner shell 111 comprises a face side with a recess 113 in which a tool can be inserted for manually rotating the inner shell 111 about the rotation axis x. In case the inner shell 111 is rotated clockwise (along M) into a second position, said wall regions (e.g. edges) E continuously engage behind the end portion 121 of the peg 12 (i.e. said narrow portion 12a is received by the slot S) and pull the peg 12 towards the locking device 11 due to an increasing thickness of the wall regions E along the slot S as can be seen from Fig. 3. This allows one to build up the proper compression force F_{c} by means of which the tibial implant 1 presses against the distal end 4 of the tibia T.

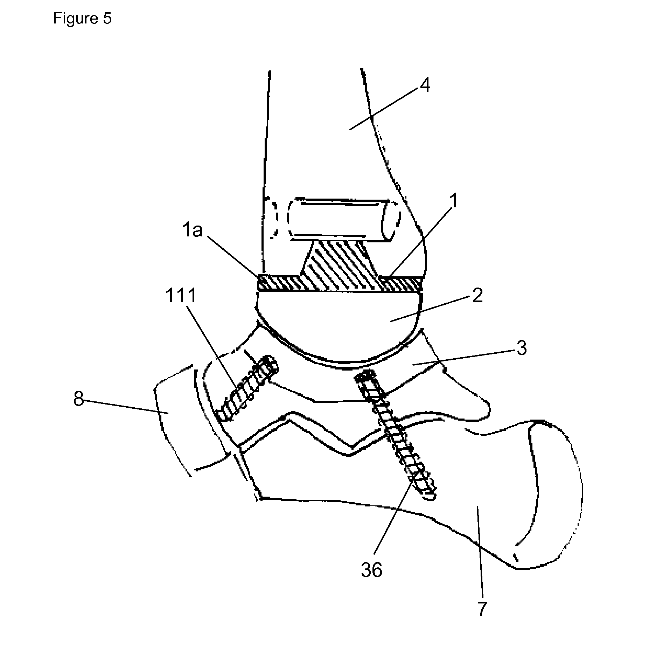

Fig. 5 shows a lateral view of a further embodiment of the invention, i.e., an extra-articular version, with the possibility of a subtalar fixation. It illustrates the tibia T, the tibial implant 1 as described in Fig.1 and Fig.2, the extra-anatomical concave intermediate implant 2 and the talar implant 3. There are possibilities of screw fixation 36 in the talus 6 and through the subtalar joint line into the calcaneus 7 or anterior (35) into the navicular bone 8.

## Claims

1. Implant system for a total ankle replacement, comprising:
- a tibial implant (1) being designed to be fastened to a distal end (4) of the tibia
(T),
**characterized by**
a separate locking device (11) being designed to be inserted into a first recess (R) of the tibia (T) extending along said distal end (4) of the tibia (T), wherein the tibial implant (1) comprises a peg (12) protruding from a base (1 a) of the tibial implant (1), which peg (12) is designed to be inserted into a second recess (R') in said distal end (4) of the tibia (T), which second recess (R') particularly communicates with the first recess (R'), and wherein when the locking device (11) is inserted into the first recess (R) and the peg (12) is inserted into the second recess (R') the locking device (11) is designed to fasten the peg (12) to the locking device (11).

2. Implant system according to claim 1, **characterized in that** when the locking device (11) is inserted into the first recess (R) and the peg (12) is inserted into the second recess (R') the locking device (11) is designed to pull the peg (12) and thereby the tibial implant (1) towards the locking device (11) so as to press the tibial implant (1) against said distal end (4) of the tibia (T).

3. Implant system according to claim 1 or 2, **characterized in that** the locking device (11) is designed to receive an end portion (121) of the peg (12) for fastening the peg (12) to the locking device (11) and/or for pulling the peg (12) towards the locking device (11).

4. Implant system according to claim 2 or 3, **characterized in that** the locking device (11) comprises a rotatable element (111) being designed to be rotated between a first and a second position about a rotation axis (x), wherein particularly upon rotation of the rotatable element (111) from the first towards the second position, the rotatable element (111) is particularly designed to engage behind said end portion (121) received by the locking device (11) and to pull said end portion (121) towards the locking device (11) so as to press the tibial implant (1) against said distal end (4) of the tibia (T).

5. Implant system according to one of the claims 3 to 4, **characterized in that** the locking device (11) comprises a tubular outer shell (11a) having a lateral aperture (O) for receiving said end portion (121) of the peg (12).

6. Implant system according to claims 4 and 5, **characterized in that** the rotatable element (111) is rotatably mounted inside said outer shell (11 a) about said rotation axis (x).

7. Implant system according to one of the claims 4 to 6, **characterized in that** the rotatable element (111) is formed as an inner shell (111), wherein particularly said inner shell (111) and said outer shell (11a) are designed to be coaxially arranged with respect to a common longitudinal axis (x) of the inner and the outer shell (111, 11a), wherein particularly said longitudinal axis (x) coincides with said rotation axis (x).

8. Implant system according to claims 3 and 7, **characterized in that** said rotatable inner shell (111) comprises a lateral aperture (O') in a circumferential wall of the inner shell (111) for receiving said end portion (121) of the peg (12), and wherein particularly for allowing the locking device (11) to receive the end portion (121) of the peg (12) said lateral aperture (O') of said rotatable inner shell (111) aligns with the lateral aperture (O) of the outer shell (11a) in said first position of the rotatable inner shell (111).

9. Implant system according to claim 8, **characterized in that** the rotatable inner shell (111) comprises a slot (S) formed in said wall of the inner shell (111), which slot (S) is connected to said aperture (O') of the inner shell and is delimited by opposing regions (E) of said wall of the inner shell (111), wherein the rotatable inner shell (111) is designed to engage behind said end portion (121) of the peg (12) with said regions (E) upon rotation from the first position into the second position, and wherein particularly the thickness of said wall regions (E) increases along the slot (S) so that upon rotation of the inner shell (111) from the first position into the second position the end portion (121) of the peg (12) is continuously pulled further into the locking device (11).

10. Implant system according to one of the claims 3 to 9, **characterized in that** said end portion (121) of the peg (12) is formed as a sphere which is connected via a narrow portion (12a) of the peg (12) to a base (12b) of the peg (12), which base (12b) of the peg (12) protrudes from the base (1a) of said tibial implant (1), wherein particularly said narrow portion (12a) is designed to be received by said slot (S) of the rotatable inner shell (111), and wherein particularly said base (12b) of the peg (12) comprises a conical shape.

11. Implant system according to one of the claims 7 to 10, **characterized in that** said inner shell (111) comprises a recess (113) in a face side of the inner shell (111) for insertion of a tool for rotating the rotatable inner shell (111) between said first and second position.

12. Implant system according to one of the claims 1 to 3, **characterized in that** the peg (12) is designed to be fastened to the locking device (11) by means of a fastening means, particularly in the form of a cone or a wedge, wherein particularly said fastening means interacts with the locking device (11) and the peg (12) such that the peg (12) is pulled towards the locking device (11) so as to press the tibial implant (1) against said distal end (4) of the tibia (T) when the fastening means fastens the peg (12) to the locking device (11).

13. Implant system according to one of the preceding claims, **characterized in that** the tibial implant (1) comprises a lower surface (14) facing away from the distal end (4) of the tibia (T), wherein particularly said lower surface (14) is flat.

14. Implant system according to one of the preceding claims, **characterized in that** the implant system further comprises a talar implant (3) which is designed to be implanted in or on the talus (6), wherein the talar implant (3) comprises an upper surface (310) facing towards the tibial implant (1), wherein particularly said upper surface (310) is a saddle-shaped upper surface.

15. Implant system according to claims 13 and 14, **characterized in that** the implant system further comprises an intermediate implant (2) being designed to be arranged between the tibial implant (1) and the talar implant (3), which intermediate implant (2) comprises an upper surface for butting against said lower surface (14) of the tibial implant (1), wherein particularly said upper surface is flat, and wherein the intermediate implant (2) comprises a lower surface (210) for butting against the upper surface (310) of the talar implant (3), wherein particularly said lower surface (210) is a saddle-shaped lower surface.
